Europäisches Patentamt

European Patent Office

Office européen des brevets

(11) Publication number: **0 078 533 B1**

(12) **EUROPEAN PATENT SPECIFICATION**

(45) Date of publication of patent specification: **21.10.92**   (51) Int. Cl.⁵: **A61K  31/045**, A61K 9/06

(21) Application number: **82110082.3**

(22) Date of filing: **02.11.82**

(54) **Medicinal composition for treating skin disorders.**

(30) Priority: **03.11.81 US 317976**
       **03.11.81 US 318076**

(43) Date of publication of application:
     **11.05.83 Bulletin  83/19**

(45) Publication of the grant of the patent:
     **21.10.92 Bulletin  92/43**

(84) Designated Contracting States:
     **AT BE CH DE FR GB IT LI LU NL SE**

(56) References cited:
     **US-A- 3 584 115**

     **CHEMICAL ABSTRACTS, vol. 94, no. 2, 12th
     January 1981, page 246, no. 7650m, Colum-
     bus, Ohio, US; SHOU-JIN WU: "Studies on
     the chemical constituents of Daqingyl
     (Clerodendron cyrtophyllum Turcz.) &
     CHUNG TS'AO YAO 1980, 11(3), 99-101**

(73) Proprietor: **Clark, Lealand L.**
     **1025 South 12th East
     Salt Lake City Utah 84105(US)**

(72) Inventor: **Clark, Lealand L.**
     **1025 South 12th East
     Salt Lake City Utah 84105(US)**

(74) Representative: **Bauer, Wulf, Dr.**
     **Wolfgang-Müller-Strasse 12
     W-5000 Köln 51 (Marienburg)(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to a medicinal composition for treating skin diseases, containing triacontanol.

US-A-3 584 115 teaches pressurized topical compositions exhibiting the property of becoming colorless under ambient temperature conditions comprising a major proportion of a liquefied normally gaseous propellant and a minor proportion of triacontanol.

According to Chemical Abstracts, vol. 94, no. 2, 12th January 1981, page 246, no. 7650m melissyl alcohol ( = triacontanol) was detected among others in the leaf of C. cyrtophyllum, a commonly used herbal medicine.

1. Herpesviruses

Herpesviruses come in 70 different varieties, but only a few are infectious to humans. The viruses infectious to humans include Herpesvirus hominis, which causes herpes simplex; Herpesvirus varicellae, which causes herpes varicella (chicken pox) and zoster (shingles); the Epstein-Barr virus, which causes mononucleosis; and cytomegalovirus, which causes fetal infections. Herpesviruses are also often responsible for fevers, hepatitis, and pneumonia-like illnesses in children and adults, especially those with lowered resistance. Additionally, eczema is caused by Herpesvirus hominis or Poxvirus officinalis, and perhaps other viruses such as Coxsackievirus.

A. Herpes Simplex

Of all the Herpesviruses, the effects of Herpesvirus hominis are by far the most commonly experienced. Herpesvirus hominis, which is responsible for herpes simplex, has two different forms: Type I and Type II. Type I causes Herpes labialis (oral herpes) in the form of cold sores and unsightly lesions around the lips or nose. Type II causes Herpes genitalis (genital herpes) in the form of sores that appear below the waist, primarily in the genital area. The two vary little with respect to the nature of their behavior and either one can take the other's place. Thus, Type II can cause a cold sore while Type I can also infect the genitals. Nevertheless, Type II is responsible for at least about eighty percent (80%) of genital herpes.

Both Types I and II can be transmitted by sexual as well as non-sexual contact; however, genital herpes is generally transmitted through sexual intercourse. A Type I infection of the genitals or a Type II infection of the mouth can occur through oral-genital contact. A cold sore virus may be transmitted when two persons kiss or by means as simple as the use of the same towel to wipe their faces. The eyes can be infected simply by rubbing them after touching an infected area. Thus, there are a variety of ways in which herpes simplex viruses I and II can be transmitted. Moreover, although not the usual case, transmission of the viruses can even occur before the syptoms of herpes simplex appear or before the infected person is aware that he or she has herpes simplex.

The symptoms of herpes simplex infections include the development of a cluster of tiny bumps or blisters, sometimes preceded or accompanied by a fever or swollen lymph glands. The blisters then crust over, and the sores disappear - usually within three weeks after the first symptoms. However, the virus remains in the body for a lifetime, hibernating in such places as the salivary glands, the nerve tissue, and the lymph nodes. After recovery from the first attack, subsequent infections may occur the next few years, until gradually the frequency of attacks diminishes. Occasionally, however, recurrences may appear over the rest of the individual's life. The reappearance of the herpes infections is then often triggered by stress, fatigue, exposure to sun, trauma, fever or menstruation.

Other complications may develop in those who are afflicted with a herpes simplex virus. If a person suffering from herpes simplex touches a sore or blister and then rubs his eyes, he may develop a serious eye infection known as herpes keratitis. Thousands of Americans annually lose their sight because of this disease.

For women, genital herpes simplex carries special risks. To begin with, genital herpes simplex has been linked to cancer of the cervix. Female herpes victims are five to seven times more likely to develop cervical cancer than non-infected females. Genital herpes simplex can also cause serious birth defects. A pregnant woman with an active genital herpes simplex infection faces a fifty percent (50%) chance of passing the disease to her baby as the child passes through the birth canal. About fifty percent (50%) of the newborn infants who develop herpes simplex die of the infection; seventy-five percent (75%) of those who survive suffer from blindness or brain damage. Fortunately, if sores are found close to the time of delivery, the doctor can perform a Caesarean-section to prevent infection of the newborn as it passes through the birth canal.

Most Americans have been exposed to the herpes simplex virus; indeed, eighty percent (80%) of the American population carries the herpes simplex virus, and antibodies against the virus have been found in up to ninety-five percent (95%) of blood samples tested. Although some people never experience symptoms, (possibly because their immune systems repulse the virus so it cannot sustain its attack), about seven out of eight people who come in sexual contact with the herpes simplex virus will contact an infection. It is estimated that from thirty (30) to seventy (70) million Americans suffer occasionally from the common form of the herpes simplex infection, that of coldsores. Moreover, it is estimated that from five (5) to twenty (20) million Americans suffer from genital herpes simplex, and that each year, half a million more Americans join these ranks.

Since there has previously existed no known effective treatment for herpes simplex, the total number of persons inflicted with herpes simplex continues to increase. Scientists have tried and rejected many different treatments for herpes such as Vitamin C, zinc, ether, and ice packs. It is evident that in the absence of a treatment for herpes simplex, this relatively new venereal disease could potentially reach epidemic proportions.

B. Eczema

Another Herpesvirus disorder which plagues many people is atopic eczema. Eczema occurs in primarily three forms: (1) the infantile form, (2) the adult form, and (3) the localized form.

The infantile form of eczema may first appear soon after birth, often by the fourth month of the infant's life. Infantile eczema is generally manifested as processes which may be red, dry, slightly scaly, cracked, and excoriated, or sometimes moist and oozing. Infantile eczema is most frequently manifested around the face, scalp, neck and diaper areas. Older children and young adults generally experience manifestation of the disease in the flexural areas and the cheeks. In fewer than half of the individuals inflicted with infantile eczema, the disease clears up by the age of four; yet even in these individuals, the disease may occur at a later age. The majority of eczema victims still experience occasional flare ups through the young adult years, up until about the age of thirty, at which time the disease usually disappears.

The adult form of eczema is generally manifested in the antecubital and popliteal areas, and in some cases around the hands, feet and face. The infected skin is generally dry, erythematous, and excoriated with bacterial crusting and redness.

The localized form of eczema, which occurs in diverse individuals, is primarily manifested around the wrists, ankles, hands, feet and ears, as well as the perianal, perivulvar, and scrotal regions.

By far the worst consequence of atopic eczema is the pruritis or itching which is associated with this disease. Those inflicted with atopic aczema often find pruritis to be a life-long companion. Any relief to be had from such intolerable itching is gratifying to say the least.

There are many factors which play a role in the occurence of atopic eczema, such as dietetic and emotional factors. Moreover, seasonal fluctuations are an important factor with atopic eczema generally becoming worse during the winter season.

One of the greatest fears of those who are inflicted with atopic eczema, is that these individuals are generally more susceptible to viral infection, and in particular, to infestation by a herpes simplex virus or a vaccinia virus. Additionally, those suffering from atopic eczema are abnormally susceptible to environmental irritants. Consequently, those inflicted with the disease are often advised to wear clothing which is soft and light; to stay away from heat sources; to take brief baths or showers not exceeding five minutes and using a minimal amount of soap; to avoid primary irritants such as paints, cleansers, solvents, chemical sprays, dusts, and the like; and sometimes to change their residence to a warm, dry temperated, unvarying climate where temperature extremes are rarely experienced.

Although there is no known cure for atopic eczema, there are various helpful treatments which all have one goal in common: to stop the intolerable itching that accompanies atopic eczema. Examples of these treatments include antiseptics, for example antibacteral cleansers such as Betadine (a registered trademark owned by Purdue Frederick Co.; Norwalk, Connecticut 06856) and Hibitaine; topical glucocorticoid creams; systemic glucocorticoids; antipruritic agents; and atibiotics. Although the atopic and systemic glucocorticoid treatments have proven most effective in treating long-continued atopic eczema, adverse topical and systemic effects are often experienced when such treatments are used. Consequently, adverse effects in those undergoing glucocorticoid treatments must be carefully monitored.

It would, therefore, be extremely desirable to provide an effective treatment for various disorders caused by the Herpesviruses, especially herpes simplex. Moreover, it would be desirable to provide an improved treatment for Herpesvirus disorders such as eczema, which is safe, having no known side effects

3

in any body locations.

## 2. Acne and Perioral Dermatitis

### A. Acne

A common skin disorder which plagues nearly all adolescents at some time or another is that of acne vulgaris (commonly referred to as "acne"). The peak incidence of acne occurs at about fourteen (14) years of age in girls and sixteen (16) years of age in boys, with the most severe cases in boys tending to occur even later. Although acne has generally been considered a teenage problem, it is a disease for those who are in their twenties and thirties as well. There are few diseases which produce more psychic trauma, maladjustment, insecurity, and feelings of inferiority than does acne.

The causes and factors influencing the development of acne are many, and generally not well understood. Acne is normally manifested in a variety of lesions, including comedomes, papules, pustules, cysts, and scars. These lesions occur in the skin areas having the greatest concentration of sebaceous glands, e.g. the face, central chest, and upper back. In severely affected subjects, lesions may also appear on the arms, back of the neck, lower back, buttocks, and thighs.

In some adolescents, acne is manifested by nothing more than a few scattered pustules and comedomes that have a relatively short duration and produce no permanent affects. Other adolescents, on the other hand, develop more extensive acne that not only persists, but also produces permanent pits and scars. The lesions which are generally responsible for the scars are the tender, red pustules and cysts. Some surveys indicate that over two percent (2%) of all high school students have severe form of acne.

Many different treatments for acne have been developed over years; however, the effectiveness of known treatments with respect to relatively difficult acne problems has been rather limited. For mild cases of acne, regular shampooing of the scalp and daily use of an abrasive soap on the acne-infected skin are typical of recommended treatments.

For treating moderate cases of acne, preparations for drying and peeling the skin area subject to comedomes, papules, and pustules are often recommended. Some typical preparations for the treatment of moderate acne include a power-base shake lotion containing resorcinol and sulfur; benzoyl peroxide; topical antibiotics such as erythromycin and clindamycin; oral antibiotics such as tetracycline; retinoic acid (vitamin A acid); as well as moderate irradiation with ultraviolet light.

For treating more severe cases of acne where pustular and cystic lesions cause scarring, a totally safe treatment has previously not been found. Some preparations which have been used to treat severe cases of acne include those containing antibiotics, glucocorticoids, or hormones. Many systemic antibiotics carry undesirable or unwarranted side effects and risks, thus restricting the use of antibiotics to those which are mild and relatively safe, such as erythromycin and tetracycline. Undesirable side effects and risks are also experienced from the use of glucocorticoids and hormones. For example, some side effects from estrogen-progesten medications (a typical hormone treatment) include thromboembolic disease, gallbladder disease, strokes, myocardial infarcts, hepatic tumors, hypertension, and endometrial cancer. Moreover, the oral ingestion or topical application of the female hormone estrogen by males may result in other undesirable side effects, such as the emergence of feminine characteristics.

Additionally, antiandrogens, ultraviolet light, cryotherapy using solid carbon dioxide or liquid nitrogen, minor surgery, chemosurgery, and even X-ray therapy have been used in the treatment of severe cases of acne. As with the other preparations or treatments, certain risks or undesirable consequences are inherent in each of these treatments as well. One important undesirable characteristic of most all of the prior art acne treatments is that the effects of these treatments are often felt throughout the body, not just the localized skin area requiring treatment.

### B. Perioral Dermatitis

Another relatively common skin disorder is perioral dermatitis. Perioral dermatitis, which primarily plagues young adult women, is less common among men and occurs occasionally in young children. This disorder is usually manifested as a localized grouping of red papules, vesicopapules, and papulopustules around the lips, mouth, chin, paranasal area, and even the upper eyelids. Perioral dermatitis lesions often burn, and exacerbations and remissions are frequent. Moreover, the lesions associated with perioral dermatitis generally worsen with pregnancy and menstruation. Other factors which aggrevate perioral dermatitis lesions include heat and perspiration. Generally, after three to six months from onset, the lesions finally disappear.

Perioral dermatitis has proven to be resistent to most therapy, although some treatments have been found to be somewhat beneficial. Some of these treatments include (1) tetracycline; (2) a 1% hydrocortisone preparation ; (3) the avoidance of all cosmetics, except for lipstick and mascara; and (4) daily cleansing with soap. The long-continued use of potent topical glucocorticoids has been known to produce the adverse effects of perioral dermatitis. Therefore, glucocorticoid treatments which are used to treat perioral dermatitis lesions must be limited in strength, the disorder can be severely complicated.

It would, therefore, be a significant advancement in the field of dermatology to provide a more effective treatment for acne, and especially for extremely severe cases of acne, which is safe, having no known side effects in any body locations. Additionally, it would be another significant advancement to provide an effective treatment for perioral dermatitis, and especially a treatment which would avoid the adverse effects of treatments such as glucocorticoid treatments.

The invention as claimed is intended to provide a remedy. It solves the problem of how to formulate a medicinal preparation for skin disorders and thus to allow an effective treatment of skin diseases, especially those mentioned above.

The active ingredient in the medicinal preparation as claimed in claim 1 is triacontanol. It has proven to be safe and has no known side effects anywhere in the body.

The advantage of this medicinal preparation is to provide an improved treatment of acne, an effective treatment of perioral dermatitis, and an effective treatment for disorders caused by a Herpesvirus, and here most especially, for herpes simplex, eczema and shingles.

A further advantage lies in the fact that a topical treatment of the said skin diseases is possible, in which only the infected skin area is exposed to the medicant treatment.

A further advantage is to be seen in a medicinal preparation, offering a novel combination of acne and perioral dermatitis treatment.

The active ingredient in the preparation is triacontanol. Triacontanol, also known as melissyl alcohol, myricyl alcohol, or hydroxytriacontane, is a straight-chain, aliphatic, thirty carbon waxy alcohol having the formula $CH_3(CH_2)_{28}CH_2OH$. Although triacontanol has been found useful for such purposes as the fertilization of crops, to the inventor's knowledge, triacontanol has not been used as the active ingredient in any medical treatment.

Experimental application of triacontanol to skin infected with acne , perioral dermatitis, or a Herpesvirus shows that triacontanol has the following advantageous qualities:

(1) it removes pain and itching; (2) it clears up lesions associated with Herpesviruses such as herpes simplex lesions; (3) it clears up acne lesions and perioral dermatitis lesions; (4) it is anti-inflammatory; (5) it restores lipid levels on the skin to normal levels (important with respect to Herpesvirus disorders such as eczema); (6) it is virus and bacteria static; and (7) it is safe and has no known side effects in any body locations.

The triacontanol preparation is prepared by simply mixing a very small quantity of triacontanol, about one hundredth of one percent (0.01%) by weight, with a medicant base until thoroughly blended. Although 0.01% triacontanol has been found sufficient for effective treatment, quantities much smaller than this are also likely to provide effective treatment.

An important consideration in the preparation is the choice of an appropriate medicant base. The selected medicant base must be compatible with the triacontanol so as to maintain it in active form for effective application. One ointment which has been employed in the present triacontanol invention is a standard USP hydrophilic ointment; a thousand grams of which contains the following compounds in the indicated amounts:

| Hydrophilic Ointment-USP | |
| --- | --- |
| Compound | Amount |
| Methylparaben | 0.25 g. |
| Propylparaben | 0.15 g. |
| Sodium lauryl sulfate | 10 g. |
| Propylene glycol | 120 g. |
| Stearyl alcohol | 250 g. |
| White petrolatum | 250 g. |
| Purified water | 370 g. |

The ingredients of hydrophilic ointment USP, which ointment is commonly available from a variety of commercial sources, are combined as follows. First, the stearyl alcohol and the white petrolatum are melted on a stream bath and warmed to about 75°C. The other ingredients are dissolved in the purified water and are also warmed to about 75°C. All ingredients are then mixed together and stirred until the mixture congeals.

It will be understood that the hydrophilic ointment disclosed above is given by way of example only, and that numerous other carrier medicants may also be suitable, such as an oleic acid ointment base. Again, it is the triacontanol, not the carrier medicant, which is the active ingredient in the preparation, the carrier medicant merely acting as a carrier for the triacontanol to provide for the effective application of the triacontanol in active form to the skin. Thus, it will be appreciated that one of the most important properties of the carrier medicant is its ability to provide sufficient contact between the active triacontanol and the skin to effectively treat the skin.

The above-described triacontanol-hydrophilic ointment preparation has been found to be effective in the treatment of disorders caused by Herpesviruses, such as herpes simplex, eczema, and zoster (shingles). In particular, the preparation has especially been found to be effective against herpes simplex and eczema.

Experimentally, the best results have been obtained in using the preparation in the treatment of herpes simplex (all types of lesions, particularly cold sores) and eczema. Although the effect of the triacontanol preparation on other Herpesvirus disorders such as chicken pox, mononucleosis, fetal infections, fevers, hepatitis, and pneumonia-like illnesses is yet unknown, it is well-anticipated that the preparation may also be effective in the treatment of these disorders.

Over twenty patients with herpes simplex lesions in the form of cold sores have been treated with the above-described triacontanol-hydrophilic ointment preparation. Upon topical application of the preparation, these patients typically found that the itching associated with the herpes simplex lesions disappeared after about 30 to 60 seconds and that the pain also disappeared after about four to eight minutes. The effectiveness of the preparation in aiding the healing process was found to vary with the age of the lesion. Typically, it was found that if a lesion was treated within four hours after its initial appearance, the lesion disappeared completely within only a few hours - commonly within about six hours For older lesions, it was found that treatment caused lesions to disappear in about one to seven days, generally reducing the normal life of the lesion by at least fifty percent (50%) . Overall, the triacontanol-hydrophilic ointment preparation decreased the normal healing time for herpes simplex lesions by up to eighty percent (80%) or more.

About 25 patient with atopic eczema were also been treated with the above-described triacontanol-hydrophilic ointment preparation. The average patient treated had experienced the symptoms ot atopic eczema for at least ten years and had suffered from the symptoms of atopic eczema during at least forty percent (40%) of the previous year. These patients found that their eczema lesions, which were four weeks old on the average, were completely healed five days after beginning treatment with the triacontanol-hydrophilic ointment preparation. This is in contrast to the three-week healing period which is generally required when the well-known cortisone cream treatments were used.

Moreover, the severe itching associated with the eczema lesions disappeared within two hours after application of the triacontanol-hydrophilic ointment preparation, whereas the cortisone creams required about two weeks to dispel the itching. Additionally, the pain and soreness associated with the lesions commonly disappeared within only a few minutes after application of the triacontanol-hydrophilic ointment preparation, as opposed to about five days for the cortisone creams. Finally, the triacontanol-hydrophilic ointment preparation decreased the normal healing time for the lesions dramatically.

The above described triacontanol-hydrophilic ointment preparation has been found to be effective in the treatment of acne and perioral dermatitis, which is somewhat related to acne. Experimentally, the best results have been obtained in using the preparation in the treatment of perioral dermatitis.

Clinical studies were conducted on about fifteen patients who, on the average, had experienced perioral dermatitis over a period of at least three years, and had experienced manifestations of the disorder at least twice a year. Normally, three to six months were required to heal the perioral dermatitis lesions of these patients. After treatment with the triacontanol-hydrophilic ointment preparation of the present invention, the time required for healing was decreased dramatically. Moreover, the itching associated with perioral dermatitis typically disappeared within only a few seconds after application of the triacontanol-hydrophilic ointment preparation to the lesions.

One of the significant advantages of using triacontanol as the active ingredient in the treatment of the present invention is that triacontanol has proven itself to be safe. Indeed, it occurs naturally in alfalfa, honey, and in the waxy portions of several edible plants. Recently, it was found that triacontanol dramatically increases yields when used as a fertilizer. A group at Michigan State University discovered that when as little as five milligrams of triacontanol were mixed with 30 to 40 gallons of water in the treatment of one acre of crops, growth was increased by an average of 12 percent. Since triacontanol is a common plant constituent, it is not unusual for one to consume enough triacontanol in one meal to treat at least an acre of crops.

Another indication that triacontanol is safe is the fact that it is found naturally in beeswax as a palmitate ester and possibly, in extremely small amounts, as the simple alcohol itself. Beeswax has been used for many years as a stiffening agent in many pharmaceutical preparations such as cerates, ointments, pastes, and petroxolins. Cerates are mostly used as dressings for inflamed skin surfaces and contain sufficient beeswax to give them a desired consistency. Moreover, many cosmetic preparations such as all-purpose creams, night creams, vanishing creams, lotions, mascaras, lipsticks, cream lip rouge, and face and body makeup contain significant amounts of beeswax. Thus, the long-time use of beeswax-containing preparations may provide a basis for the belief that triacontanol is safe when applied topically.

Although found in many preparations presently on the market, it is unclear why, if any triacontanol is present in beeswax as the free alcohol, it is not active against the a.m. skin diseases. First of all, since it is present in beeswax as the palmitate, it is thought that triacontanol is not active when in the form of a palmitate ester. Moreover it is conjectured that even if a very small amount of triacontanol is present as a free alcohol in beeswax, perhaps the amount is too small to be effective or perhaps the environment imposed by the other constituents of beeswax in some way prohibits the triacontanol from having any effect. This supposed inhibitive effect could be due in part to hydrophilic interaction between triacontanol molecules in the beeswax environment. Additionally, the inactivity of any triacontanol in beeswax might also possibly be explained by the inability of the skin to effectively absorb any triacontanol from the beeswax environment. Furthermore, it is also possible that very close contact between the triacontanol and the monomolecular cell wall of the virus is required for effective treatment. Such close contact could very well be inhibited by the beeswax environment surrounding any triacontanol which might be present. Whatever the reason for the ineffectiveness of beeswax in treating acne, perioral dermatitis, and herpesvirus disorders, for purposes of the present invention, it will be appreciated that the choice of an appropriate carrier medicant for the triacontanol, such as the hydrophilic ointment described hereinabove, is very important.

The foregoing descriptions are to be considered in all respects as illustrative. The scope of the invention is, therefore, indicated by the appended claims rather than by the foregoing description. All changes which come within the meaning and range of equivalency of the claims are to be embraced within their scope.

**Claims**

1. Use of a medicinal composition containing triacontanol, excluding compositions containing a liquefied, normally gaseous aerosol propellant, for the preparation of a medicament for treating skin disorders.

2. Medicinal composition for treating skin disorders containing triacontanol in an amount of less than one percent (in weight), excluding compositions containing a liquefied, normally gaseous aerosol propellant.

3. Medicinal composition as claimed in claim 2 comprising
   - a carrier medicant, preferably a hydrophilic ointment, and
   - triacontanol dispersed throughout the carrier medicant.

4. Medicinal composition as claimed in claim 3, in which the hydrophilic ointment contains the following compounds:

| Compound | Amount in weight % |
| --- | --- |
| Methylparaben | 0,025 % |
| Propylparaben | 0,015 % |
| Sodium lauryl sulfate | 1 % |
| Propylene glycol | 12 % |
| Stearyl alcohol | 25 % |
| White petrolatum | 25 % |
| Purified water | 37 % |

5. Medicinal composition as claimed in claim 2, 3 or 4 in which the percentage (in weight) of triacontanol is between 0,1 % and 0,001 %, said percentage preferably amounting to 0,01 %.

6. Medicinal composition as claimed in one of the claims 2 to 5, characterised by its use in treating skin infected with a herpesvirus, especially herpes simplex virus, an eczema virus and/or zoster virus, acne and/or perioral dematitis.

7. Medicinal composition as claimed in one of the claims 2 bis 6, characterised by a preparation to be topically applied to the skin areas infected.

**Patentansprüche**

1. Verwendung einer medizinischen Zusammensetzung, die Triacontanol enthält, mit Ausnahme von Zusammensetzungen, die ein verflüssigtes, normalerweise gasförmiges Aerosol-Treibmittel enthalten, für die Herstellung eines Medikaments zur Behandlung von Hautkrankheiten.

2. Medizinische Zusammensetzung für die Behandlung von Hautkrankheiten, die Triacontanol in einer Menge von weniger als einem Gewichtsprozent enthält, ausschließlich Zusammensetzungen, die ein verflüssigtes, normalerweise gasförmiges Aerosol-Treibmittel enthalten.

3. Medizinische Zusammensetzung nach Anspruch 2, enthaltend
   - einen Medikamentenhilfsstoff, vorzugsweise eine hydrophile Salbe, und
   - Triacontanol, das im Medikamentenhilfsstoff durch und durch verteilt ist.

4. Medizinische Zusammensetzung nach Anspruch 3, bei dem die hydrophile Salbe folgende Bestandteile aufweist:

| Bestandteil | Menge in Gewichtsprozent |
|---|---|
| Methylparaben | 0,025 % |
| Propylparaben | 0,015 % |
| Natriumlaurylsulfat | 1 % |
| Propylenglykol | 12 % |
| Stearylalkohol | 25 % |
| weißes Vaselin | 25 % |
| gereinigtes Wasser | 37 % |

5. Medizinische Zusammensetzung nach Anspruch 2, 3 oder 4, bei dem der Gewichtsanteil von Triacontanol zwischen 0,1 % und 0,001 % liegt, wobei der Gewichtsanteil vorzugsweise 0,01 % beträgt.

6. Medizinische Zusammensetzung nach einem der Ansprüche 2 bis 5, gekennzeichnet durch ihre Verwendung in der Behandlung von Haut, die infiziert ist mit einem Herpesvirus, vorzugsweise Herpessimplexvirus, einem Ekzem-Virus und/oder Zostervirus, Akne und/oder periorale Dermatitis.

7. Medizinische Zusammensetzung nach einem der Ansprüche 2 bis 6, gekennzeichnet durch eine Zubereitung, die lokal auf die befallenen Hautbereiche auftragbar ist.

**Revendications**

1. Utilisation d'une composition médicamenteuse renfermant du triacontanol, à l'exclusion des compositions contenant un propulseur d'aérosol liquéfié et normalement gazeux, pour la préparation d'un médicament destiné au traitement d'affections cutanées.

2. Composition médicamenteuse pour le traitement d'affections cutanées, composition qui contient du triacontanol en une quantité d'au mois 1 % (en poids), à l'exclusion des compositions contenant un propulseur d'aérosol liquéfié et normalement gazeux.

3. Composition médicamenteuse selon la revendication 2 qui contient :

- un excipient pour médicament, de préférence une pommade hydrophile, et
- du triacontanol dispersé dans toute la masse de l'excipient.

4. Composition médicamenteuse selon la revendication 3, composition dans laquelle l'excipient hydrophile contient les composés suivants :

| Composé | Quantité en % en poids |
|---|---|
| méthylparabène | 0,025 % |
| propylparabène | 0,015 % |
| sulfate de lauryle et de sodium | 1 % |
| propylène-glycol | 12 % |
| alcool stéarylique | 25 % |
| vaseline blanche | 25 % |
| eau purifiée | 37 %. |

5. Composition médicamenteuse selon l'une quelconque des revendications 2, 3 et 4, dans laquelle le pourcentage (en poids) du triacontanol est compris entre 0,1 et 0,001 %, et est de préférence égal à 0,01 %.

6. Composition médicamenteuse selon l'une quelconque des revendications 2 à 5, caractérisée en ce qu'elle est utilisée dans le traitement de la peau infectée par un virus herpétique, en particulier par le virus Herpes simplex, un virus d'eczéma et/ou un virus de zona, de l'acné et/ou de la dermatite péri-orale.

7. Composition médicamenteuse selon l'une quelconque des revendications 2 à 6, caractérisée en ce qu'il s'agit d'une préparation destinée à être appliquée localement sur les régions de la peau qui sont infectées.